(19) Europäisches Patentamt
European Patent Office
Office européen des brevets

(11) EP 1 498 102 A2

(12) # DEMANDE DE BREVET EUROPEEN

(43) Date de publication:
**19.01.2005 Bulletin 2005/03**

(51) Int Cl.[7]: **A61K 7/00**, A61K 7/06

(21) Numéro de dépôt: **04291769.0**

(22) Date de dépôt: **12.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **16.07.2003 FR 0308679**

(71) Demandeur: **L'OREAL**
**75008 Paris (FR)**

(72) Inventeurs:
- **Rollat-Corvol, Isabelle**
**75017 Paris (FR)**
- **Samain, Henri**
**91570 Bievres (FR)**

(74) Mandataire: **Wattremez, Catherine**
**L'OREAL - D.I.P.I.**
**25-29 Quai Aulagnier**
**92600 Asnières (FR)**

(54) **Composition comprenant au moins un polymère conducteur et au moins un propulseur, et procédé la mettant en oeuvre**

(57) L'invention concerne une composition comprenant, dans un milieu cosmétiquement acceptable, (a) au moins un propulseur, (b) au moins un polymère conducteur de préférence comprenant au moins une unité répétitive de type des anilines, pyrroles, thiophènes ou bisthiophènes, furanes, para-phénylène-sulfures, para-phénylène vinylènes, indoles, amides aromatiques, hydrazides aromatiques, azométhines aromatiques, esters aromatiques particuliers.

Elle concerne de plus un procédé mettant en oeuvre une telle composition, ainsi que l'utilisation de cette composition pour conférer un effet optique aux fibres kératiniques.

EP 1 498 102 A2

## Description

L'invention concerne une composition comprenant, dans un milieu cosmétiquement acceptable, au moins un polymère conducteur et au moins un propulseur. Elle concerne également un procédé de traitement de fibres kératiniques mettant en oeuvre la composition précitée. Elle a enfin pour objet l'utilisation d'une telle composition afin d'apporter un effet optique aux fibres kératiniques.

La présente invention a plus particulièrement trait au domaine des traitements de fibres kératiniques, de préférence humaines telles que notamment les cheveux.

La plupart des traitements appliqués aux fibres kératiniques, comme notamment les procédés de coloration, de décoloration ou encore de déformation permanente ont des conséquences importantes sur les caractéristiques des fibres, et notamment sur leur brillance. Ainsi, à la suite de traitements répétés, il n'est pas rare de constater que les fibres traitées deviennent plus ou moins ternes, et cela malgré les améliorations qui ont été apportées aux procédés mis en oeuvre.

Afin de compenser ces effets négatifs, et dans le but d'apporter de la brillance aux cheveux, il est connu d'utiliser par exemple des substances hydrophobes lubrifiantes, telle que des huiles ou des cires organiques ou des silicones.

Toutefois, l'effet de brillance obtenu manque d'intensité et donne en général aux fibres, un aspect artificiel.

De plus, ces compositions apportent aux fibres, un toucher gras ou collant non souhaité.

Enfin, ces composés peuvent avoir des conséquences non souhaitées lorsqu'ils sont présents dans des compositions de coloration. On a effectivement pu constater que la montée du colorant se faisait de manière moins efficace si la composition tinctoriale comprenait ces composés. Cela peut notamment se traduire par des colorations moins puissantes, moins tenaces.

La présente invention a donc pour but de proposer des compositions qui apportent aux fibres kératiniques traitées un aspect brillant sans les inconvénients rencontrés avec les compositions classiques.
Par ailleurs, dans certains cas, la composition selon l'invention peut apporter de la couleur aux fibres kératiniques sur lesquelles elle est appliquée.

La présente invention a ainsi pour premier objet une composition comprenant, dans un milieu cosmétiquement acceptable,

(a) au moins un propulseur, et
(b) au moins un polymère conducteur.

L'invention concerne de plus un procédé de traitement des fibres kératiniques humaines, et plus particulièrement des cheveux, avec une composition comprenant lesdits polymères conducteurs, consistant à appliquer sur les fibres sèches ou humides, la composition selon l'invention, puis à sécher ou à laisser sécher les fibres.

L'invention a de même pour objet l'utilisation d'une composition comprenant au moins un propulseur et au moins un polymère conducteur pour conférer un effet optique aux fibres kératiniques.

En effet, la composition selon l'invention apporte aux fibres, et de façon uniforme, notamment une brillance plus intense, plus naturelle, et plus esthétique qu'avec les moyens de l'art antérieur.

Par ailleurs, lorsque les polymères conducteurs présents dans la composition selon l'invention absorbent dans le spectre visible, on obtient simultanément un effet optique, comme de la brillance, et de la couleur.

Enfin, les fibres traitées avec la composition selon l'invention présente un toucher doux, non gras.

Mais d'autres caractéristiques et avantages de l'invention apparaîtront plus clairement à la lecture de la description et de l'exemple qui suivent.

Dans ce qui va suivre et à moins d'une indication différente, les bornes d'un domaine de valeurs sont comprises comme faisant partie de ce domaine.

Au sens de la présente invention, le terme effet optique recouvre des effets de brillance, de couleur, métallique, goniochromatique, moiré, fluorescent, thermochrome, électrochrome.

Par ailleurs, et plus particulièrement, il est à noter que la brillance correspond à l'intensité lumineuse réfléchie sous un angle $\alpha$ lorsque la mèche de cheveux est éclairée sous un angle $-\alpha$. L'angle $\alpha$ classiquement utilisé pour mesurer cette réflexion spéculaire, autrement dit la brillance, est égal à 20°. Cet apport de brillance peut être mesuré par utilisation d'un brillance mètre comme il est par exemple décrit dans la norme ISO 2813-1994 de l'AFNOR (août 1994, rectificatif février 1997).

<u>Polymères conducteurs</u>

Selon la présente invention, on entend par "polymère conducteur" une structure moléculaire dans laquelle le ou les monomères présentent une forte délocalisation électronique et dont la disposition dans le squelette du polymère

permet aux orbitales π de se recouvrir. Cette caractéristique chimique se traduit par un phénomène de conduction électrique qui s'accompagne ou non d'un phénomène d'absorption dans le spectre UV-visible, voire dans l'infrarouge.

Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.

Les polymères conducteurs mis en oeuvre dans le cadre de la présente invention sont des polymères conducteurs solubles ou dispersibles dans le milieu cosmétique approprié à l'application.

On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.

De façon préférée, les polymères conducteurs mis en oeuvre dans le cadre de la présente invention sont des polymères conducteurs solubles ou dispersibles dans un milieu aqueux, avantageusement dans l'eau.

On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Il est à noter que de manière avantageuse, ces polymères ne nécessitent pas l'emploi d'un agent dispersant.

De préférence, les polymères conducteurs se présentent sous une forme soluble dans le milieu de la composition.

De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.

La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.

Dans cette configuration la conductivité de l'échantillon exprimée en S/cm est donnée par l'expression suivante:

$$\sigma = (K \times I)/(U \times e)$$

Avec:

K coefficient dépendant de la position des contacts sur la surface de l'échantillon.
Lorsque les pointes sont alignées et équidistantes, K est égal à : $\Pi/\log(2)$
I : valeur du courant injecté exprimé en ampères
U : valeur de la tension mesurée exprimée en volts
e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.

Selon un mode de réalisation particulier, les polymères conducteurs présents dans la composition sont choisis parmi les polymères comprenant au moins une unité répétitive de formules suivantes :

<u>les anilines de structure (I) suivante</u>:

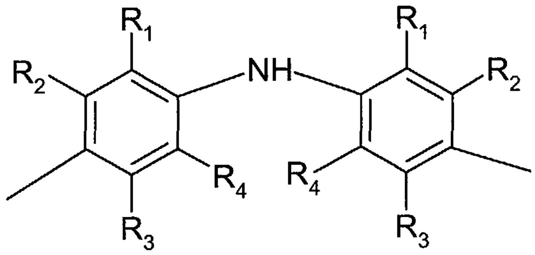

(I)

les pyrroles de structure (IIa) et (IIb) suivantes:

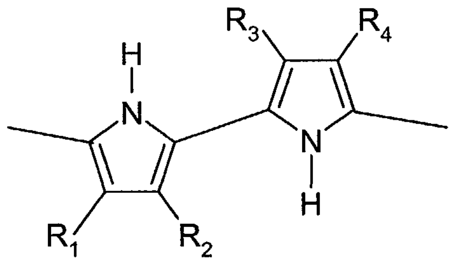

(IIa)

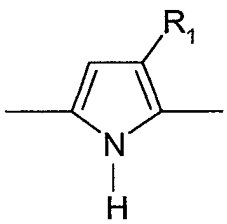

(IIb)

les thiophènes ou bisthiophènes de formules (IIIa), (IIIb) et (IIIc) suivantes:

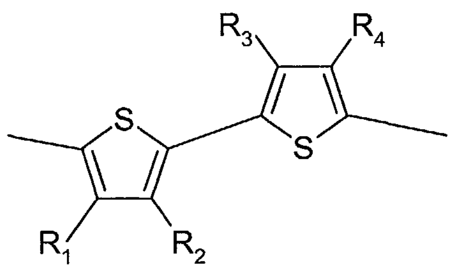

(IIIa)

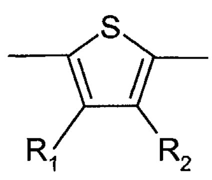

(IIIb)

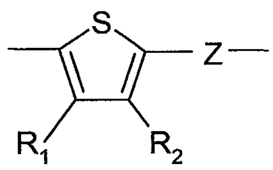

(IIIc)

les furanes de formule (IV) suivante:

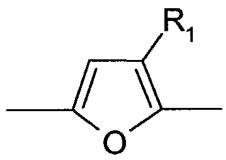

(IV)

les para-phénylène-sulfure de structure (V) suivante:

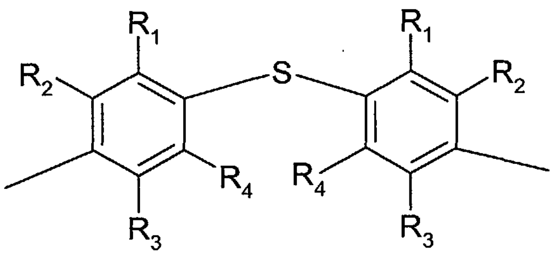

(V)

les paraphénylène vinylène de formule (VI) suivante:

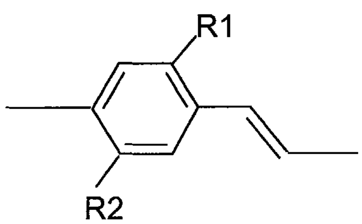

(VI)

les indoles de formule (VII) suivante:

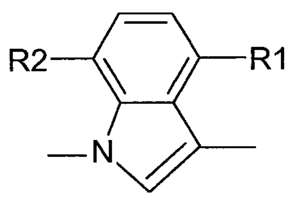

(VII)

les amides aromatiques de formules suivantes (VIIIa), (VIIIb), (VIIIc),(VIIId):

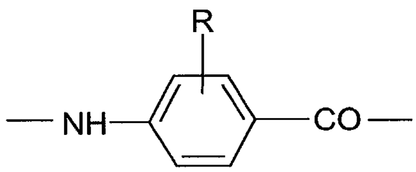

(VIIIa)

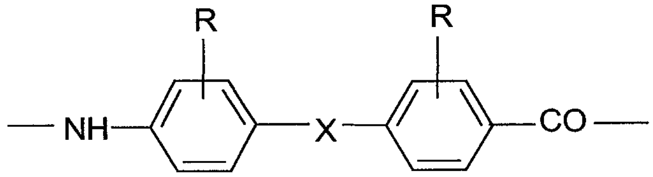

(VIIIb)

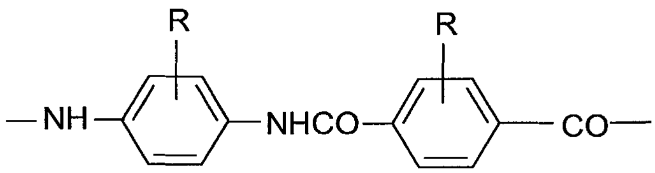

(VIIIc)

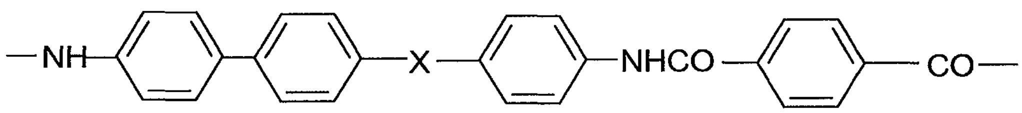

(VIIId)

les hydrazides aromatiques de formules (IXa), (IXb) et (IXc) suivantes:

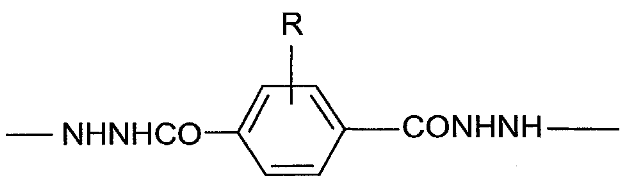

(IXa)

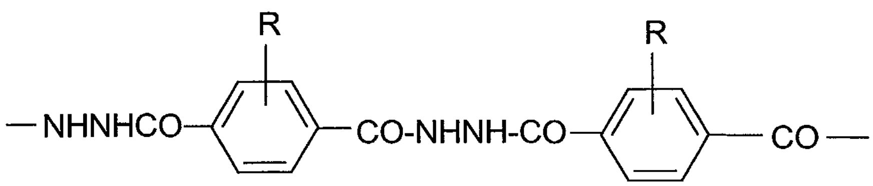

(IXb)

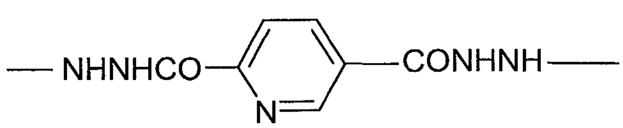

(IXc)

les azométhines aromatiques de formules (Xa), (Xb) et (Xc) suivantes:

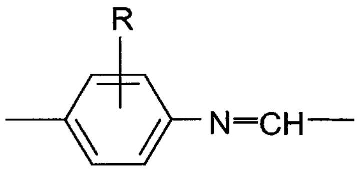

(Xa)

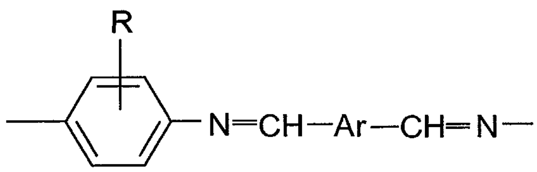

(Xb)

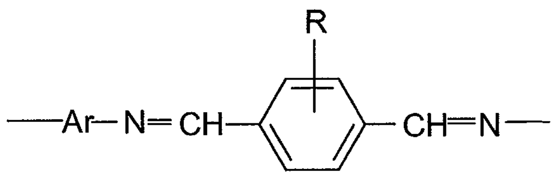

(Xc)

les esters aromatiques de formules (XIa), (XIb) et (XIc) suivantes:

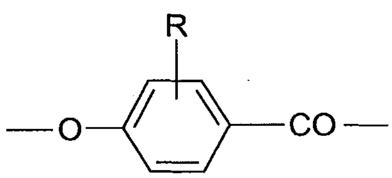

(XIa)

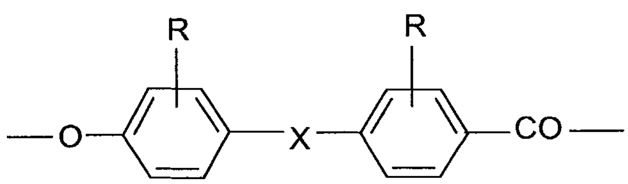

(XIb)

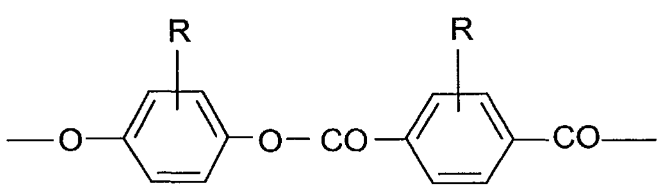

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, -$SO_2$-,

-N=N-, $-C(CH_3)_2-$, $-CH_2-$, -CH=CH-, -CH=N-;
Z= -CH=CH- ou -C≡C-.

Plus particulièrement. Ar représente au moins un radical choisi parmi les suivants :

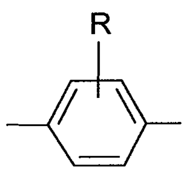 , 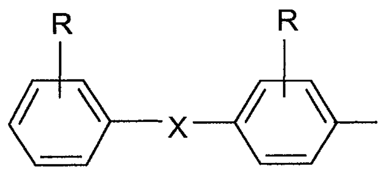 , 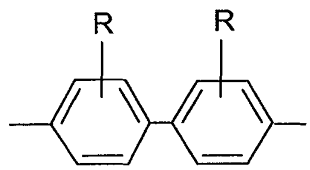 .

Par groupement solubilisant, on entend au sens de la présente invention, un groupement qui assure la solubilisation de ladite molécule dans le milieu cosmétique, de façon que le polymère présente un caractère conducteur après séchage de la composition.

Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.

Les groupements solubilisants sont de préférence choisis dans le groupe formé par :

- un radical carboxylique (-COOH), carboxylate (-COO-M+ avec M représentant un métal alcalin comme le sodium, le potassium, un métal alcalino-terreux, une amine organique telle que une amine primaire, secondaire ou tertiaire, une alcanolamine, un acide aminé),
- un radical sulfonique ($-SO_3H$), sulfonate ($-SO_3^- M^+$, M ayant la même définition que ci-dessus),
- un radical amine primaire, secondaire, tertiaire,
- un radical ammonium quaternaire tel $-NR'3^+$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un acide organique, tel que les acides acétique ou lactique, par exemple.

En outre, il est à noter que lesdits radicaux solubilisants peuvent être reliés au cycle par l'intermédiaire d'un groupement espaceur tel que par exemple un radical -R"-, -OR"-, -OCOR"- ou encore -COOR"- avec R" représentant un radical alkyle, linéaire ou ramifié en $C_1$-$C_{20}$, comprenant éventuellement un ou plusieurs hétéroatomes, tels que l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$ $-(CH_2)_3SO_3H$, $-O(CH_2)_3SO_3H$, $-O(CH_2)_3N(CH_2CH_3)_2$, $-[(CH_2)_2O]xCH_2CH_2O$ $-[(CH_2)_2O]xCH_2CH_2OCH_3$ avec x nombre moyen compris entre 0 et 200.

Le nombre d'unités répétitives du polymère n varie habituellement de 5 à 10000, notamment de 5 à 1000, plus particulièrement de 10 à 1000 et de préférence de 20 à 700.

Plus particulièrement, le polymère conducteur est tel qu'au moins un radical parmi R, R1 à R4 désigne un groupement solubilisant.

Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.

De préférence, le polymère conducteur est soluble dans le milieu de la composition.

Les polymères conducteurs présents dans la composition selon l'invention sont bien connus de l'homme de l'art et décrits notamment dans l'ouvrage "Handbook of organic conductive molecules and polymers" - Wiley 1997-New York, Vol 1, 2, 3, mais aussi dans la revue Can. J. Chem. Vol 64, 1986.
Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general approach to tuning the properties of functionalized polythiophenes : The oxidative polymerization of monosubstituted bithiophenes" ; dans l'article tiré de la revue Macromolecules 1998, 31, pages 933-936, par les mêmes auteurs "Highly

conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.
Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'3^{+}$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeCl_3$); par polycondensation de thiophène dihalogéné catalysée par des complexes de nickel ou de palladium (ex. : $NiCl_2(dppe)_2$) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B ) ou de type A-B (réaction de plusieurs monomères de type A-X-B)) ; par couplage de Stille (formation de liaison carbone-carbone en présence d'une catalyse à base de Pd-type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant -CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant-C≡C- peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de $Mo(CO)_6$).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3^{+}$ Z- avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.
Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5

et 10 % en poids, par rapport au poids total de la composition.

Comme indiqué auparavant, la composition selon l'invention comprend au moins un propulseur.

Il est à noter que le propulseur peut ou non être soluble dans la composition.

Plus particulièrement, le propulseur est choisi parmi les hydrocarbures en C3-C5, tels que le n-butane, le propane, l'isobutane, le pentane ; les hydrocarbures fluorés ou chlorés ; le gaz carbonique ; le protoxyde d'azote ; le diméthyléther ; l'azote ; l'air comprimé. On peut aussi utiliser des mélanges de propulseurs.
De préférence, on utilise le diméthyl éther.

La teneur en propulseur est de manière avantageuse comprise entre 5 et 90 % en poids par rapport au poids total de la composition dans le dispositif aérosol et, plus particulièrement, à une concentration comprise entre 10 et 60 % en poids, par rapport au poids total de la composition dans le dispositif aérosol.

Par ailleurs, le milieu cosmétiquement acceptable de la composition est de l'eau ou un mélange d'eau et d'un ou plusieurs solvants organiques acceptables dans le domaine.

Parmi les solvants utilisables, on peut citer plus particulièrement, les alcools en C1-C4, tels que l'alcool éthylique, l'alcool isopropylique, les alcools aromatiques comme l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

L'homme du métier peut sans difficulté déterminer la composition appropriée du solvant présent dans la composition. Cependant de préférence, le solvant comprend au moins 50 % en volume en alcool en C2-C4, de préférence au moins 70 % en volume d'alcool en C2-C4.

La composition selon l'invention est de plus conditionnée dans un récipient aérosol approprié mis sous pression à l'aide du propulseur précité.

La composition peut de plus comprendre au moins un colorant direct.

Plus particulièrement, ledit colorant direct est de nature non ionique, cationique ou anionique.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, ou les colorants naturels (comme le henné, la camomille), seuls ou en mélanges.

Lorsqu'ils sont présents, le ou les colorants directs représentent de préférence de 0,0005 à 12 % en poids du poids total de la composition, et encore plus préférentiellement de 0,005 à 6 % en poids du poids total de la composition.

La composition selon l'invention peut de même comprendre au moins un tensioactif, qui peuvent être choisis parmi des tensioactifs anioniques, amphotères, non ioniques, zwittérioniques et cationiques, ainsi que leurs mélanges.

Parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensioactifs non-ioniques du type hydroxypropyléther.

Lorsque la composition comprend un ou plusieurs tensioactifs, leur teneur est habituellement de moins de 30% en poids, et de préférence comprise entre 0,5 et 10% en poids, par rapport au poids de la composition.

La composition cosmétique peut encore comprendre une quantité efficace d'autres agents, par ailleurs antérieurement connus dans le traitement des fibres kératiniques humaines, tels que des polymères fixants, des agents épaississants, des polymères filmogènes, des polymères coiffants, des agents anti-oxydants, des parfums, des agents dispersants, des agents de conditionnement dont notamment des polymères cationiques ou amphotères, des agents opacifiants, des filtres UV, des agents conservateurs, des céramides, des pseudocéramides, les vitamines ou provitamines comme le panthénol, des polymères associatifs non-ioniques, anioniques, amphotères ou cationiques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition selon l'invention est donc conditionnée de manière à obtenir une laque ou une mousse.

La composition qui vient d'être décrite est en général appliquée sur les fibres kératiniques, sèches ou humides, puis on sèche ou on laisse sécher les fibres.

Il peut aussi être avantageux de les mettre en forme au moment du séchage.
Cette opération de séchage a lieu en général dans une gamme de température comprise entre 20 et 120°C, de préférence entre 20 et 80°C.

L'exemple suivant illustre l'invention sans pour autant présenter un caractère limitatif.

**EXEMPLE**

**Synthèse de poly(thiophène-3- acide acétique)**

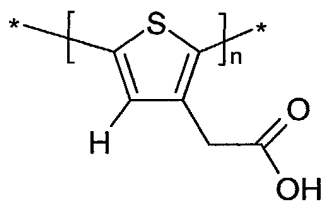

Mode opératoire

Préparation du aolymère : poly(thiophène-3-acétate d'éthyle)

Dans un schlenk sous argon, on introduit 25 ml de chloroforme sec, on dégaze puis on introduit les réactifs : 25g de thiophène-3-acétate d'éthyle (14,7 mmol)

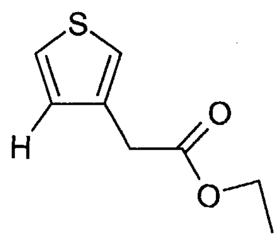

et 1 g de FeCl3 (6,15 mmol).

Le mélange est agité pendant 24 heures sous argon à 50°C.

Le polymère poly(thiophène-3-acétate d'éthyle) est alors précipité dans l'heptane.

Le polymère est ensuite dissous dans une solution de tétrahydrofuranne

Caractérisation par infra rouge:

Bande du C=O : 1719 $cm^{-1}$ ; bandes du $CH_2$, $CH_3$ = 2979 $cm^{-1}$ , 2934 cm-1 et disparition de la bande CH à 3102 $cm^{-1}$ présente dans le monomère.

Hydrolyse du polymère: poly(thiophène-3-acétate d'éthyle) pour former le poly(thiophène-3- acide acétique). Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).

Le polymère est ensuite filtré et lavé à plusieurs reprises par de l'eau distillée afin d'éliminer les traces de catalyseur.

Caractérisation du polymère Infra-rouge:

Bande du C=O : 1740 $cm^{-1}$ ; COO 1580 cm-1 ; OH (bande large 3000-3500 $cm^{-1}$)

Neutralisation du polymère poly(thiophène-3- acide acétique):

Le polymère poly(thiophène-3- acide acétique) (2g) est dissous dans le tétrahydrofuranne (30g) et neutralisé à raison de 1 mol d'hydroxyde de sodium par mole d'acide carboxylique.

L'eau (30 g) est ensuite additionnée.

Le tétrahydrofuranne est évaporé.

Il est ainsi obtenu une solution aqueuse 6% de poly(thiophène-3- acide acétique) sous forme d'un sel de sodium.

| **Formulation comprenant le polymère et procédé la mettant en oeuvre** | |
|---|---|
| Poly(thiophène-3-acide acétique) | 1g |
| Aminométhyl propanol qs | pH 7 |
| Eau qs | 50 g |
| Alcool éthylique | 15 g |
| Diméthyléther | 35 g |

Le bidon est équipé d'une valve munie d'un tube plongeur et d'un bouton poussoir muni d'une buse.

La composition est vaporisée à environ 20 cm des cheveux pendant environ 5 secondes et apporte un effet brillant aux cheveux.

## Revendications

1. Composition comprenant, dans un milieu cosmétiquement acceptable,

   (a) au moins un propulseur, et
   (b) au moins un polymère conducteur.

2. Composition selon la revendication 1 dans laquelle le polymère conducteur comprend au moins une unité répétitive de formules suivantes :

   les anilines de structure (I) suivante:

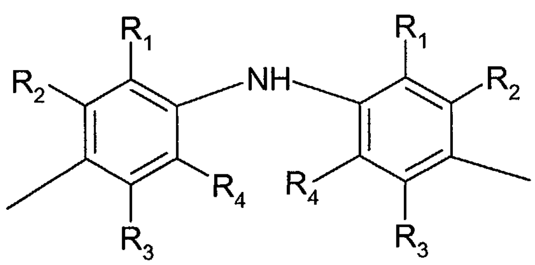

(I)

   les pyrroles de structure (IIa) et (IIb) suivantes:

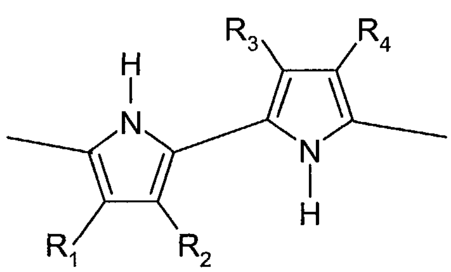

(IIa)

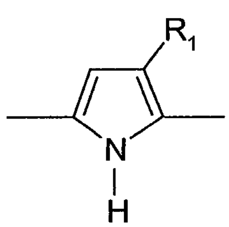

(IIb)

les thiophènes ou bisthiophènes de formules (IIIa), (IIIb) et (IIIc) suivantes:

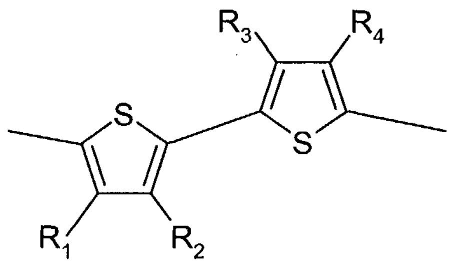

(IIIa)

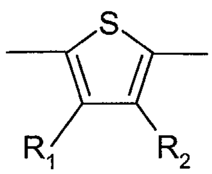

(IIIb)

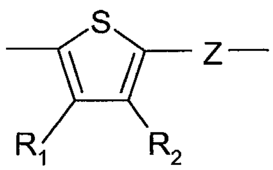

(IIIc)

les furanes de formule (IV) suivante:

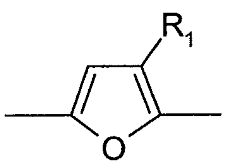

(IV)

les para-phénylène-sulfure de structure (V) suivante:

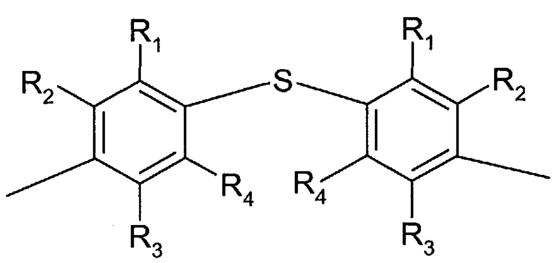

(V)

les paraphénylène vinylène de formule (VI) suivante:

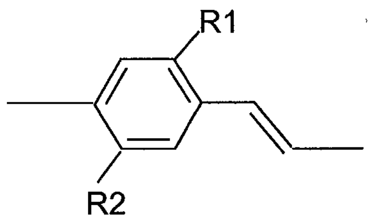

(VI)

les indoles de formule (VII) suivante:

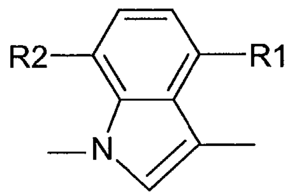

(VII)

les amides aromatiques de formules suivantes (VIIa), (VIIb), (VIIIc), (VIIId):

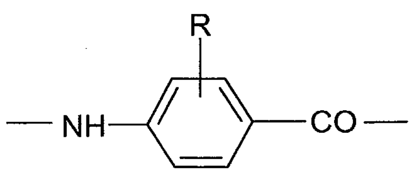

(VIIIa)

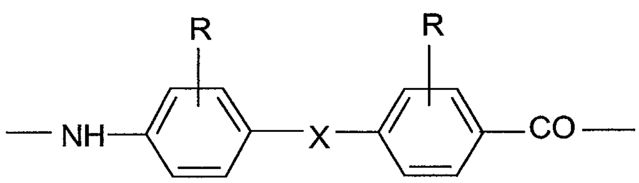

(VIIIb)

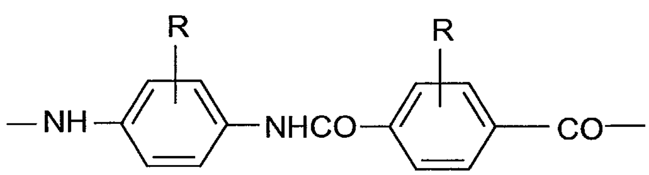

(VIIIc)

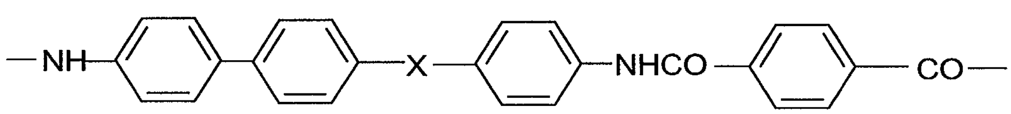

(VIIId)

les hydrazides aromatiques de formules (IXa), (IXb) et (IXc) suivantes:

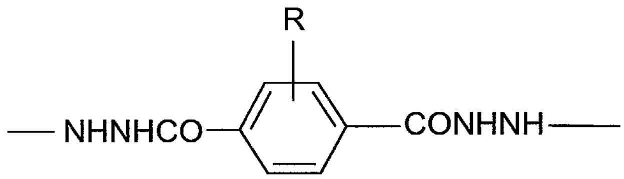

(IXa)

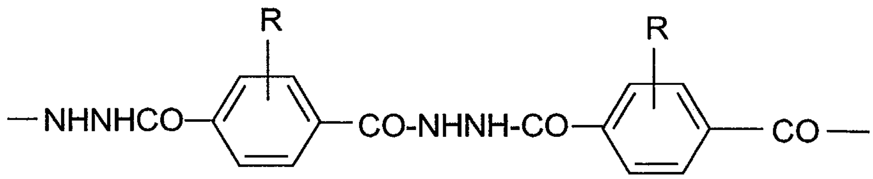

(IXb)

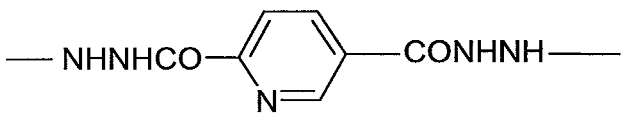

(IXc)

les azométhines aromatiques de formules (Xa), (Xb) et (Xc) suivantes:

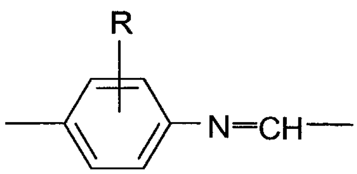

(Xa)

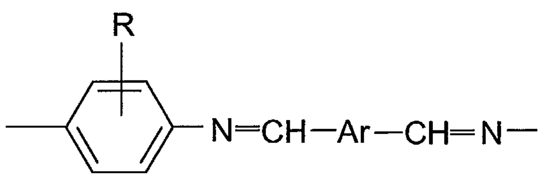

(Xb)

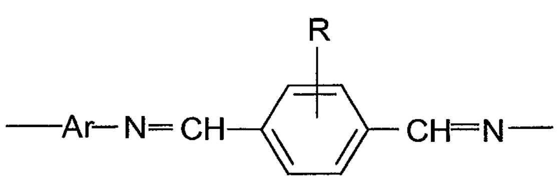

(Xc)

les esters aromatiaues de formules (XIa), (XIb) et (XIc) suivantes:

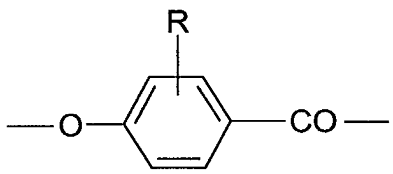

(XIa)

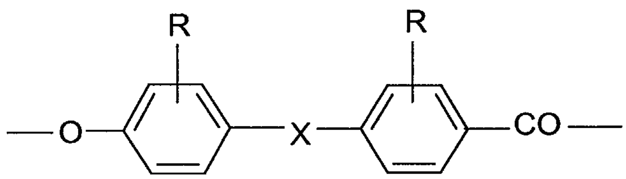

(XIb)

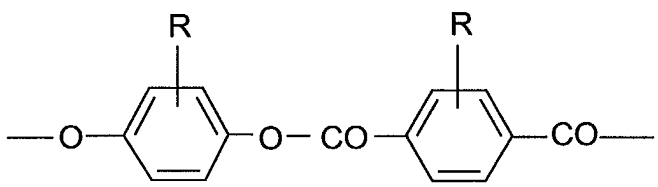

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant, étant entendu qu'au moins un radical parmi R, $R_1$ à $R_4$ désigne un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N-;
Z = -CH=CH- ou -C≡C-.

**3.** Composition selon la revendication 2, **caractérisée en ce que** les groupements solubilisants sont choisis dans le groupe formé par :

- un radical carboxylique (-COOH), carboxylate ($-COO-M^+$ avec M représentant un métal alcalin, un métal alcalino-terreux, une amine organique, une alcanolamine, un acide aminé),
- un radical sulfonique ($-SO_3H$), sulfonate ($-SO_3^- M^+$, M ayant la même définition que ci-dessus),
- un radical amine primaire, secondaire, tertiaire,
- un radical ammonium quaternaire tel $-NR'_3{}^+$ Z- avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alcène en $C_2$-$C_3$.

**4.** Composition selon l'une quelconque des revendications précédentes, **caractérisée en ce que** les groupements solubilisants sont reliés au cycle par l'intermédiaire d'un groupement espaceur tel que par exemple un radical -R''-, -OR''-, -OCOR''- ou -COOR''- avec R'' représentant un radical alkyle, linéaire ou ramifié en $C_1$-$C_{20}$, comprenant éventuellement un ou plusieurs hétéroatomes.

**5.** Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]xCH_2CH_2OH$, $-[(CH_2)_2O]xCH_2CH_2OCH_3$ avec x nombre moyen compris entre 0 et 200.

**6.** Composition selon l'une quelconque des revendications 1 à 3, **caractérisée en ce que** le polymère se présente

sous une forme soluble dans le milieu.

7. Composition selon l'une quelconque des revendications 1 à 4, **caractérisée en ce que** le polymère comporte au moins un groupement solubilisant par unité répétitive.

8. Composition selon l'une quelconque des revendications précédentes, **caractérisée en ce que** les groupements solubilisants sont choisis parmi les groupements acide carboxylique ; acide sulfonique ; les radicaux amine tertiaire ; ammonium quaternaire tels que $-NR'_3{}^+$ Z- avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

9. Composition selon l'une des revendications précédentes, **caractérisé en ce que** le polymère conducteur correspond aux formules (IIIa), (IIIb) ou (IIIc), dans lesquelles au moins un radical $R_1$ à $R_4$ de la formule (IIIa) ou $R_1$ ou $R_2$ des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en $C_1$-$C_{20}$, le ou les autres radicaux représentant un atome d'hydrogène.

10. Composition selon l'une quelconque des revendications précédentes, **caractérisée en ce que** le ou les polymères conducteurs sont présents dans des proportions d'au moins 0,001 % en poids par rapport au poids total de la composition.

11. Composition selon l'une quelconque des revendications précédentes, **caractérisée en ce que** le ou les polymères conducteurs sont présents dans des proportions d'au plus 50 % en poids par rapport au poids total de la composition.

12. Composition selon l'une quelconque des revendications précédentes, **caractérisée en ce que** le teneur en polymère conducteur représente 0,1 à 50 % en poids par rapport au poids total de la composition.

13. Composition selon l'une quelconque des revendications précédentes, **caractérisée en ce que** le propulseur est choisi parmi les hydrocarbures en C3-C5, les hydrocarbures fluorés ou chlorés, le gaz carbonique, le protoxyde d'azote, le diméthyléther, l'azote, l'air comprimé.

14. Composition selon la revendication précédente, **caractérisée en ce que** la teneur en propulseur est comprise entre 5 et 90 % en poids par rapport au poids total de la composition dans le dispositif aérosol et, plus particulièrement, à une concentration comprise entre 10 et 60 % en poids, par rapport au poids total de la composition dans le dispositif aérosol.

15. Composition selon l'une quelconque des revendications précédentes, **caractérisée en ce que** le milieu cosmétiquement acceptable est l'eau ou un mélange eau-solvant.

16. Composition selon la revendication 15, **caractérisée en ce que** le ou les solvants sont choisis parmi des alcools, des glycols, des éthers de glycol, des polyols et les éthers de polyols, des polyéthylèneglycols, le polypropylèneglycol et leurs mélanges.

17. Composition selon la revendication précédente, **caractérisée en ce que** le solvant le solvant comprend au moins 50 % en volume en alcool en C2-C4, de préférence au moins 70 % en volume d'alcool en C2-C4.

18. Composition selon l'une quelconque des revendications précédentes, **caractérisée en ce qu'**elle comprend au moins un colorant direct.

19. Composition selon la revendication précédente, **caractérisée en ce que** le colorant direct est choisi parmi les colorants directs non ioniques, cationiques ou anioniques.

20. Composition selon l'une des revendications 18 ou 19, **caractérisée en ce que** la teneur en colorant direct représente de 0,0005 à 12 % en poids, par rapport au poids de la composition tinctoriale.

21. Procédé de traitement des fibres kératiniques humaines et plus particulièrement les cheveux, **caractérisé en ce**

**qu'**il consiste à appliquer sur les fibres sèches ou humides la composition selon l'une quelconque des revendications précédentes, et à sécher ou à laisser les fibres ainsi traitées.

**22.** Utilisation d'une composition comprenant au moins un polymère conducteur, au moins un propulseur, selon l'une quelconque des revendications 1 à 20, pour conférer un effet optiques à des fibres kératiniques.

**23.** Utilisation selon la revendication précédente, **caractérisée en ce que** l'effet optique est un effet de brillance.